# EUROPEAN PATENT SPECIFICATION

(11) **EP 3 503 947 B1**
(45) Date of publication and mention of the grant of the patent: **13.11.2024**
(21) Application number: 17749741.9
(22) Date of filing: 14.08.2017
(51) Int. Cl.: A61M 5/31, A61M 5/36, G01C 9/10, G01C 9/28, A61M 5/315

(54) **ORIENTATION INDICATOR FOR A MEDICAMENT DELIVERY DEVICE AND A MEDICAMENT DELIVERY DEVICE COMPRISING THE ORIENTATION INDICATOR**
AUSRICHTUNGSINDIKATOR FÜR EINE MEDIKAMENTENABGABEVORRICHTUNG UND TRAGBARE MEDIKAMENTENABGABEVORRICHTUNG MIT DEM AUSRICHTUNGSINDIKATOR
INDICATEUR D'ORIENTATION POUR UN DISPOSITIF D'ADMINISTRATION DE MÉDICAMENT ET DISPOSITIF D'ADMINISTRATION DE MÉDICAMENT COMPRENANT L'INDICATEUR D'ORIENTATION

(30) Priority: 25.08.2016 EP 16185634
(43) Date of publication of application: 03.07.2019
(73) Proprietor: SHL Medical AG, 6302 Zug (CH)
(72) Inventor: HOLMQVIST, Anders, 139 40 Värmdö (SE)
(86) International application number: PCT/EP2017/070638
(87) International publication number: WO 2018/036860

(56) References cited:
- EP-A1- 3 175 876
- WO-A1-03/081175
- WO-A1-2010/019456
- WO-A1-2010/125153
- WO-A1-2012/164397
- US-A- 6 004 285
- US-A1- 2009 235 744
- US-A1- 2013 153 206
- US-A1- 2016 213 856

## Description

### TECHNICAL FIELD

The present invention relates to an orientation indicator for a medicament delivery device, and more particularly to an orientation indicator for informing a user of a medicament delivery device that a proper orientation of the device is attained.

### BACKGROUND

The development of medicament delivery devices has become more and more directed towards self-medication, i.e. to let the patient administer a medicament to him- or herself in an easy, safe and reliable way. There are different types of medicaments that can be stored for a long time and that are packaged in cartridges or the like, containing a ready-to-use medicament in liquid state. There are also other types of medicaments that are stored in separate compartments. An agent (e.g. lyophilized, powdered or concentrated liquid) is stored in one compartment of the container, and a diluent (e.g. water, dextrox solution or saline solution) is stored in a separate compartment. These types of medicaments cannot be pre-mixed and stored for a long time because the medicament agent is unstable and can be degraded and will lose its effect quickly. Hence, the patients have to perform the mixing shortly before administration. During the mixing step, a significant amount of air needs to be evacuated from the powder compartment as it is filled with diluent. This air needs to escape through a delivery member, e.g. a needle, a mouthpiece or a nozzle, of the device. At the same time it is important not to spill any of the liquid medicament during the mixing procedure.

Other types of delivery devices comprise a container with a pre-mixed solution. Still, some air may need to be evacuated before use. The device needs to be "primed". Priming is necessary to avoid injecting air into the bloodstream and to make sure that a correct dosage is administrated.

In both types of delivery devices, the devices must be oriented with its delivery member pointing upwards. Conventionally, air is evacuated by the patient tapping the device with a finger, to make sure that any air in the container is collected at the top, near the opening of the container. Thereafter, the container is pressurized, such as by pushing the piston rod forward, until some of the liquid medicament appears at an opening of the delivery member, e.g. at the tip of an injection needle. The patient therefore needs to be taught and trained on how to handle the medicament delivery device before usage.

There are a number of prior art devices that register patient handling of medicament delivery devices. One example is US2015/0246179, wherein sensors are used to detect the progress of an injection process, such as temperature, dosage and orientation. A controller determines the progress and provides a notification thereof, both directly, by beeping, and indirectly, by sending data to a smartphone app.

Document WO 2010/125153 A1 discloses a pen-type injector which has a priming detection facility to identify when the injector is inverted.

The disclosed solution is unnecessarily complicated for many applications. The present invention provides a simple and robust orientation indicator that is also reliable and easy to use.

### SUMMARY

An object of the present invention is to provide an orientation indicator for a medicament delivery device, wherein the drawbacks of the state of the art devices are remedied.

In the present application, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

Further, the term "longitudinal", with or without "axis", refers to a direction or an axis through the device or components thereof in the direction of the longest extension of the device or the component.

The term "lateral", with or without "axis", refers to a direction or an axis through the device or components thereof in the direction of the broadest extension of the device or the component.

In a similar manner, the terms "radial" or "transversal", with or without "axis", refers to a direction or an axis through the device or components thereof in a direction generally perpendicular to the longitudinal direction, e.g. "radially outward" would refer to a direction pointing away from the longitudinal axis.

Also, if nothing else is stated, in the following description wherein the mechanical structure of the device and the mechanical interconnection of its components is described, the device is in an initial non-activated or non-operated state.

As stated above, the aim of the present invention is to provide a simple and robust orientation indicator that is also reliable and easy to use.

According to a main aspect of the invention it is characterized by the features of the independent patent claim. Further advantageous features are the subject of the dependent claims.

According to a main aspect of the invention it is characterised by an orientation indicator for a medicament delivery device, which orientation indicator comprises a support element having a proximal end and a distal end, an activation member, movable, in relation to the support element, between a first position and a second position, an electrical circuit comprising a signal switch, and a signalling element connected to the electrical circuit, wherein the activation member may move under gravitational force when the support element is tilted to actuate the signal switch, such that a signal may be generated by the signalling element.

A movable element, which moves under the influence of gravity, is a robust and simple solution. The element may slide, roll, pivot or fall between the first and the second positions, as long as it is able to interact with, i.e. to close, or to open, the signal switch.

According to another aspect of the invention the activation member is made of a conductive material, such that actuation of the signal switch, by the activation member, closes or opens the signal switch.

Conductivity is a further advantage in that the movable element may directly close or open the electrical circuit by bridging or opening the gap between the poles of the signal switch. No additional mechanical or electrical component needs to be involved in actuating the switch.

According to another aspect of the invention the activation member is slidably movable between the first position and the second position.

A slidable activation member may have any suitable shape and may be formed to provide a small contact surface with a substrate on which it slides. The slidable activation member may be made of a material that provides a low coefficient of friction with the substrate on which it slides.

According to another aspect of the invention the activation member is rollably movable between the first position and the second position.

A rollable activation member may be ball-shaped, cylindrical, or have any other shape that allows a rolling motion.

According to another aspect of the invention the activation member is a ball and wherein the ball and the signal switch constitute a tilt ball switch.

According to another aspect of the invention the activation member is made of a liquid conductor.

According to another aspect of the invention the liquid conductor is mercury and wherein the liquid conductor and the signal switch constitute a mercury switch.

Mercury switches are generally known in other fields of technology. Nowadays, however, mercury is often avoided due to its poisonous and environmentally polluting properties.

According to another aspect of the invention the activation member is held fixed in the first position until released by a manually operable locking member.

During normal handling of a medicament delivery device, the device is often tilted in various directions. In order to avoid activating the signalling element, the activation member may be held fixed in the first position until it is time to use the device. The manually operable locking member may be of any configuration known in the art. It may be operated by pushing or sliding a button, turning a knob, etc.

According to another aspect of the invention the electrical circuit further comprises a manually operable second switch, and wherein the signal is generated by the signalling element when the signal switch and the second switch are actuated.

In a similar fashion to the manually operable locking member, the second switch serves to prevent the signalling element from being activated prematurely, i.e. to prevent the signalling device from activating accidentally before the device is to be used. The second switch may be opened or closed to set the electrical circuit in an active state, before or after the signal switch is actuated. The second switch may be actuated by turning a knob, operating a button, etc.

According to another aspect of the invention the signal generated by the signalling element is an audible signal and/or a tactile signal and/or a visual signal.

The signalling element may display a visible light, or a colour, or a symbol, or the like.

The signalling element may generate a sound, which may sound positive or negative, depending on the orientation in which the electrical circuit and the signalling element are designed to be activated.

The signalling element may generate a tactile sensation, such as a vibration, which is distributed in the device in which the orientation indicator is arranged.

According to another aspect of the invention the second position is located proximally, relative to the first position, such that a negative signal is generated by the signalling element, indicating an incorrect orientation.

The activation member moves under the influence of gravity. Therefore, if the second position is proximal of the first position it means that the signalling element is activated when the proximal part of the orientation indicator is lower than the distal part, meaning that a negative signal will be generated. A negative signal may for instance be a red light, a dissonant noise or an unpleasant vibration.

According to another aspect of the invention the second position is located distally, relative to the first position, such that a positive signal is generated by the signalling element, indicating a correct orientation.

As opposed to the situation above, if the second position is distal of the first position it means that the signalling element is activated when the proximal part of the orientation indicator is higher than the distal part, meaning that a positive signal will be generated. A positive signal may for instance be a green light, a harmonic melody or a soft vibration.

According to another aspect of the invention the support element is attachable to a medicament delivery device.

The orientation indicator may be designed as a packaged module that may be attached to an outer part of a medicament delivery device. In this way, the orientation indicator may be used for many types of medicament delivery devices, which do not need to be further adapted to accommodate the orientation indicator.

According to another aspect of the invention the support element is integrated in a medicament delivery device.

By integrating the orientation indicator in a medicament delivery device, a more compact design may be achieved. The orientation indicator may thereby be arranged on the inside of the device, such that visual signals may be viewed through a window.

According to another aspect of the invention the electrical circuit also comprises a control unit and a communication unit capable of wirelessly communicating orientation data with an external device, which orientation data is retrieved from the control unit.

A further aspect of the invention relates to a medicament delivery device comprising an orientation indicator according to any of the previous aspects of the invention. Alternatively, the orientation indicator can be used for a training device or a trainer, which is simulating the medicament delivery device and used for training of users prior their use of a real medicament delivery device. The trainer may comprise an orientation indicator.

These and other features and advantages of the present invention will become apparent from the following detailed description and the drawings.

### BRIEF DESCRIPTION OF THE DRAWINGS

The figures below disclose an embodiment of the invention for illustrational purposes only. In particular, the disclosure within the figures is not meant to limit the range of protection of the invention. The embodiment shown may be modified in different ways within the scope of the claims.
- Fig. 1: a perspective view of a medicament delivery device comprising an orientation indicator according to the invention
- Fig. 2: an exploded perspective view of the orientation indicator and the medicament delivery device
- Fig. 3: perspective view of an orientation indicator attachable to a medicament delivery device
- Figs 4-5: perspective views of the orientation indicator in use

### DETAILED DESCRIPTION

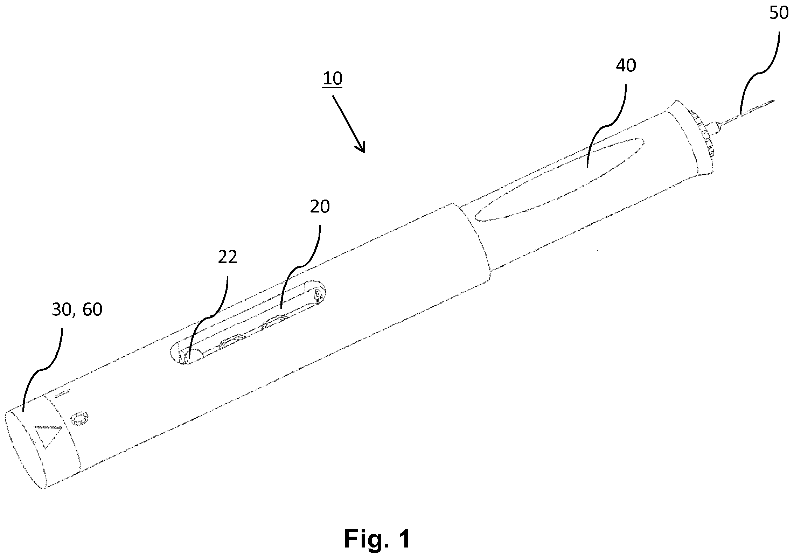

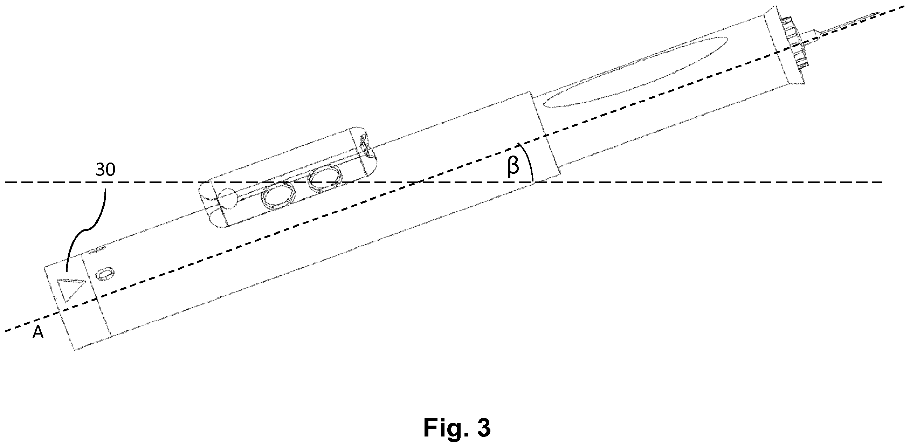

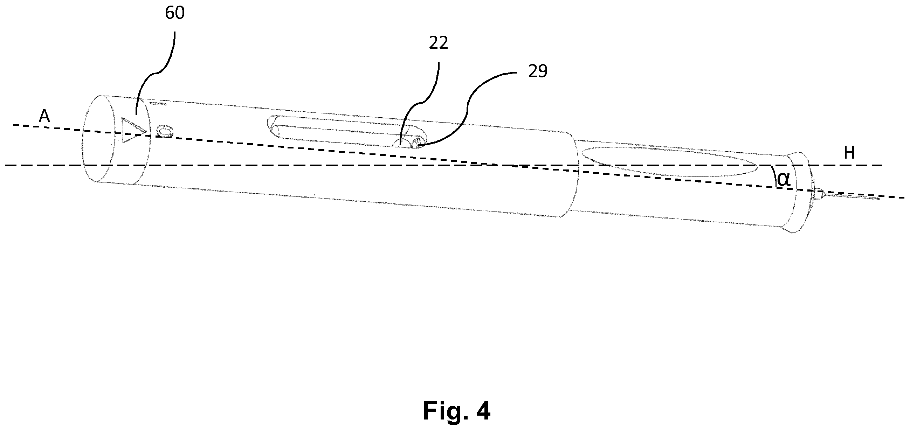

Fig. 1 shows a perspective view of a medicament delivery device 10 comprising an orientation indicator according to the presently claimed invention. A support element 20, having a proximal end and a distal end, is shown. The support element 20 is generally elongated along a longitudinal axis A (Fig. 3 and 4) of the medicament delivery device 10. An activation member 22 is movable in relation to the support element 20, between a first position and a second position. The activation member 22 may be accommodated in the support element 20. The first position and the second position may be defined as end points of longitudinal movement of the activation member 22 in relation to the support element 20. The activation member 22 may move between the first position and the second position, under influence of gravitational force, when the orientation indicator is tilted in relation to a horizontal plane.

Fig. 1 further shows a delivery member 50 through which a medicament of the medicament delivery device 10 may be expelled. The delivery member may be a needle, or a nozzle, or a mouthpiece, etc. A container (not shown), holding the medicament to be expelled, may be visible through a window 40.

The support element 20 of the orientation indicator may be integrated in a medicament delivery device as shown in Fig. 1. The support element 20 may also be a separate module that may be attached to a medicament delivery device 10 to provide an orientation indication function (Fig. 3) to almost any existing delivery device.

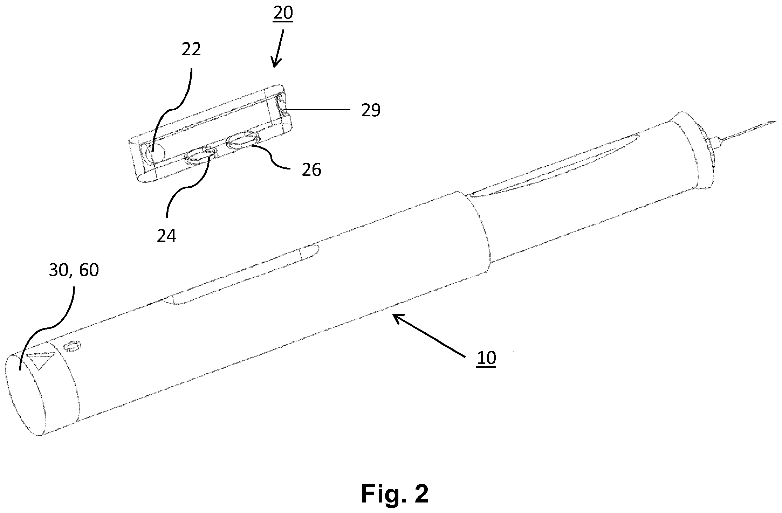

Fig. 2 displays the support element 20 as comprising an elongated compartment comprising the activation member 22. The support element further comprises a power source 24 and a signalling element 26. The power source 24 may for instance be a battery. The signalling element 26 may be sound-generating, such as a speaker, and/or visual, such as a light emitter or a display. The signalling element may also be tactile, such as a piezoelectric element configured to generate mechanical vibrations. The support element 20 comprises an electrical circuit and a signal switch 29. The signal switch 29 may be actuated by the activation member 22 by movement of the activation member 22 relative to the support element 20. The signal switch 29 may be actuated by the activation member 22 either opening or closing the signal switch 29. The signal switch is defined to be located at the second position of the activation member 22.

When integrated in a medicament delivery device 10, the support element 20 may be generally regarded as comprised in a body of the medicament delivery device 10. Accordingly, the various components comprised by the support element 20 may be distributed throughout the medicament delivery device, as appropriate, to be properly adapted to the various functions and features of both the medicament delivery device 10 and of the orientation indicator.

By integrating the orientation indicator in a medicament delivery device, a more compact design may be achieved. The orientation indicator may thereby be arranged on the inside of the device, such that visual signals may be viewed through a window.

An advantage of the orientation indicator as a separate module is that it may be used for most kinds of already existing medicament delivery devices.

The activation member 22 is preferably made of a conductive material, e.g. metal, such that actuation of the signal switch 29, by the activation member 22, closes the signal switch such that an electrical current from the power source 24 activates the signalling element 26. In an alternative embodiment the electrical circuit may be designed such that the signal switch 29 is opened by movement of the activation member 22.

Using conductivity as an activating means is advantageous in that the movable element may in itself directly close or open the electrical circuit by bridging or opening the gap between the poles of the signal switch. No additional mechanical or electrical component needs to be involved in activating the switch.

The activation member 22 may be designed in different ways to achieve the preferred characteristics, e.g. speed of movement, friction, etc. The activation member 22 may be slidably movable between the first position and the second position. A sliding motion is relatively slow and needs a certain tilting angle to overcome the friction between the activation member 22 a sliding surface of the support element 20.

The slidable activation member may have any suitable shape and may be formed to provide a large or a small contact surface with the sliding substrate on which it slides. The slidable activation member may be made of a material that provides a required coefficient of friction with the substrate on which it slides.

The activation member may alternatively be rollably movable between the first position and the second position. A rollable activation member 22 may be ball-shaped, cylindrical, or have any other shape that allows a rolling motion. Friction is negligible and movement may be initiated by only a small tilt of the orientation indicator.

In the illustrated embodiment, the activation member 22 is a ball, and the ball and the signal switch 29 may constitute a tilt ball switch.

The activation member 22 may be a liquid conductor. The liquid conductor may be mercury, and the liquid conductor and the signal switch 29 may constitute a mercury switch.

Mercury switches are generally known in other fields of technology. Nowadays, however, mercury is often avoided due to its poisonous and environmentally polluting properties. However, other liquid conductors are also conceivable.

The activation member 22 may be held fixed in the first position until released by a manually operable locking member 30. During normal handling of a medicament delivery device, the device is often tilted in various directions, which might cause the orientation indicator to accidentally generate signals. In order to avoid activating the signalling element, the activation member may therefore be held fixed in the first position until it is time to use the device. The manually operable locking member may be of any configuration known in the art. It may be operated by pushing or sliding a button, or turning a knob, etc. As illustrated in the figures, the manually operable locking member 30 may be moved between a locking position in which the activation member 22 is prevented from moving, and a released position in which the activation member 22 is released for movement. In the figures, the locking position is marked "0" and the released position is marked "I". The locking mechanism that fixes the activation member 22 may be selected from any suitable locking mechanism known to the skilled person.

Alternatively, the electrical circuit may comprise a manually operable second switch, and wherein the signal is generated by the signalling element 26 when both the signal switch 29 and the second switch 60 are actuated.

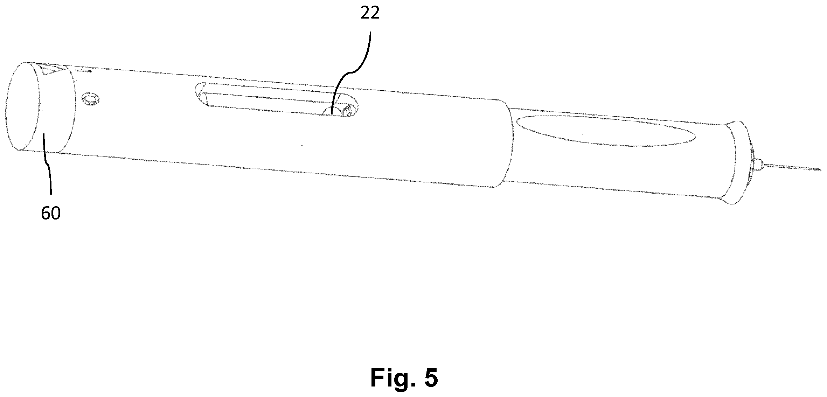

In a similar fashion to the manually operable locking member 30, the second switch 60 serves to prevent the signalling element 26 from being activated prematurely, i.e. to prevent the signalling element 26 from accidental activation before the device is to be used. The second switch 60 may be opened or closed to set the electrical circuit in an active state, before or after the signal switch 29 is actuated. The second switch 60 may be actuated by turning a knob, operating a button, etc. The second switch 60 shown in Figs 4 and 5 may be turned between an inactive state "0" and an active state "I".

The signal generated by the signalling element 26 may be an audible signal, a tactile signal or a visual signal. The generated signal may also be a combination of those. The visual signal may be visible light, or a colour, or a symbol, or the like. The tactile signal may be a tactile sensation, such as a vibration, which is distributed from the support element 22, throughout the device in which the orientation indicator is arranged.

The signal generated by the signalling element 26 may be designed to be perceived as either positive or negative, depending on the orientation in which the electrical circuit and the signalling element are designed to be activated. A light may be green (positive) or red (negative). A sound may be harmonious (positive) or dissonant (negative). A tactile signal may be soft (positive) or hard and intense (negative).

A correct orientation of a medicament delivery device, comprising the orientation indicator, during a mixing or a priming operation is to have the delivery member 50 pointing upwards from a horizontal plane. Consequently, the orientation indicator must be configured to signal either a good orientation or a bad orientation. The electrical circuit may also be configured to activate the signalling element 26 when the activation member 22, moving under the force of gravity, either closes or opens the signal switch 29. As explained earlier, the signal switch 29 is located at the second position of the activation member 22. Thus, four configurations are conceivable:
a) The delivery member 50 is pointed downward. A negative signal is generated when the activation member 22 closes the signal switch 29 of the electrical circuit, i.e. the second position is located proximally of the first position. The activation member 22 moves downward to the second position to close the signal switch 29.
b) The delivery member 50 is pointed downward. A negative signal is generated when the activation member 22 opens the signal switch 29 of the electrical circuit, i.e. the second position is located distally of the first position. The activation member 22 moves downward to the first position to open the signal switch 29.
c) The delivery member 50 is pointed upward. A positive signal is generated when the activation member 22 closes the signal switch 29 of the electrical circuit, i.e. the second position is located distally of the first position. The activation member 22 moves downward to the second position to close the signal switch 29.
d) The delivery member 50 is pointed upward. A positive signal is generated when the activation member 22 opens the signal switch 29 of the electrical circuit, i.e. the second position is located proximally of the first position. The activation member 22 moves downward to the first position to open the signal switch 29.

Figs 4-5 serve to exemplify the use of the orientation indicator. The illustrated case is applicable for alternative configurations (a) or (d) above. The medicament delivery device 10 comprises a second switch 60, which is in the inactive state "0". The medicament delivery device 10 is tilted such that the proximal end comprising the delivery member 50 is at an angle α below the horizontal plane H. When compared to the situation shown in Fig. 3, where the delivery member of the medicament delivery device 10 is tilted an angle β above the horizontal plane H, the activation member 22 of Fig. 4 has moved to the second position and has closed the signal switch 29. Since the medicament delivery device 10 is equipped with the manually operable second switch 60, no signal is generated since the second switch is in the inactive position "0".

When the user sets the second switch 60 to the active position "I" (Fig. 5), the signalling element 26 will be activated and may immediately generate a negative signal if configured as configuration (a) because the signal switch 29 is closed and the second switch 60 is in the active position. In an alternative embodiment the orientation indicator will generate a positive signal, if configured as configuration (d), as soon as the delivery member is tilted above the horizontal plane, because the activation member 22 will move towards the first position and thereby open the signal switch 29 while the second switch 60 is in the active position.

The electrical circuits that provide the above-described functions are readily available to the skilled person and do not require any further explanation, except to note that in case of activation by opening a switch, a detection circuit may need to be provided as part of the electrical circuit, in order to detect the opening of the switch and to at least partly activate the signalling element 26.

With information technology, it is also conceivable to configure the electrical circuit to also comprise a control unit and a communication unit capable of wirelessly communicating orientation data with an external device, which orientation data is retrieved from the control unit. In this way, information about the use of the device may be stored and or communicated to e.g. a physician who may then be able to use the information to teach and to improve the patient's usage of the device.

## Claims

1. An orientation indicator for a medicament delivery device (10) having a longitudinal axis (A) and a delivery member (50); the medicament delivery device (10) being configured to be oriented with its delivery member (50) pointing upwards during mixing or priming, wherein the orientation indicator comprises
- a support element (20) having a proximal end and a distal end and generally elongated along the longitudinal axis (A) of the medicament delivery device (10),
- an activation member (22) movable in relation to the support element (20), between a first position and a second position,
- an electrical circuit (28) comprising a signal switch (29),
- a signalling element (26) connected to the electrical circuit (28),
wherein the activation member (22) is movable under gravitational force when the support element (20) is tilted, to actuate the signal switch (29), such that a signal is generated by the signalling element (26) informing a user that a proper orientation of the device (10) has been attained for mixing or priming.

2. The orientation indicator for a medicament delivery device (10) according to claim 1, wherein the activation member (22) is made of a conductive material, such that actuation of the signal switch (29), by the activation member (22), closes or opens the signal switch (29).

3. The orientation indicator for a medicament delivery device (10) according to claim 2, wherein the activation member (22) is slidably movable between the first position and the second position.

4. The orientation indicator for a medicament delivery device (10) according to claim 2, wherein the activation member (22) is rollably movable between the first position and the second position

5. The orientation indicator for a medicament delivery device (10) according to claim 2, wherein the activation member (22) is made of a liquid conductor and wherein the liquid conductor may flow between the first position and the second position.

6. The orientation indicator for a medicament delivery device (10) according to claim 5, wherein the liquid conductor is mercury and wherein the liquid conductor and the signal switch (29) are comprised in a mercury switch.

7. The orientation indicator for a medicament delivery device (10) according to any of the claims 1-6, wherein the activation member (22) is held fixed in the first position until released by a manually operable locking member (30).

8. The orientation indicator for a medicament delivery device (10) according to any of the claims 1-6, wherein the electrical circuit (28) further comprises a manually operable second switch (60), and wherein the signal is generated by the signalling element (26) when the signal switch (29) and the second switch (60) are actuated.

9. The orientation indicator for a medicament delivery device (10) according to claim 1, wherein the signal generated by the signalling element (26) is an audible signal and/or a tactile signal and/or a visual signal.

10. The orientation indicator for a medicament delivery device (10) according to claim 1, wherein the support element (20) is attachable to the medicament delivery device (10).

11. The orientation indicator for a medicament delivery device (10) according to claim 1, wherein the support element (20) is integrated in the medicament delivery device (10).

12. The orientation indicator for a medicament delivery device (10) according to any of claims 1-6, wherein the electrical circuit (28) also comprises a control unit and a communication unit capable of wirelessly communicating orientation data with an external device, which orientation data is retrieved from the control unit.

13. The orientation indicator for a medicament delivery device (10) according to any of claims claim 1-12, wherein the orientation indicator is designed as a packaged module to be attached to an outer part of a medicament delivery device (10).

14. The orientation indicator for a medicament delivery device (10) according to claim 9, wherein the signalling element (26) is able generate a positive signal, indicating a correct orientation or a negative signal, indicating an incorrect orientation.

15. A medicament delivery device (10) comprising the orientation indicator according to any of claims 1-14.

## Patentansprüche

1. Ausrichtungsindikator für eine Arzneimittelabgabevorrichtung (10), die eine Längsachse (A) und ein Abgabeteil (50) aufweist; wobei die Arzneimittelabgabevorrichtung (10) konfiguriert ist, um mit ihrem Abgabeteil (50) während eines Mischens oder eines Vorbereitens nach oben zeigend ausgerichtet zu sein, wobei der Ausrichtungsindikator umfasst:
- ein Stützelement (20), das ein proximales und ein distales Ende aufweist und im Allgemeinen entlang der Längsachse (A) der Arzneimittelabgabevorrichtung (10) verlängert ist,
- ein Aktivierungsteil (22), das in Bezug auf das Stützelement (20) zwischen einer ersten Position und einer zweiten Position bewegbar ist,
- eine elektrische Schaltung (28), umfassend einen Signalschalter (29),
- ein Signalisierungselement (26),das mit der elektrischen Schaltung (28) verbunden ist,
wobei das Aktivierungsteil (22) unter Schwerkraft bewegbar ist, wenn das Stützelement (20) geneigt wird, um den Signalschalter (29) zu betätigen, derart, dass durch das Signalelement (26) ein Signal, das einen Benutzer darüber informiert, dass eine richtige Ausrichtung der Vorrichtung (10) zum Mischen oder Vorbereiten erreicht wurde, erzeugt wird.

2. Ausrichtungsindikator für eine Arzneimittelabgabevorrichtung (10) nach Anspruch 1, wobei das Aktivierungsteil (22) aus einem leitfähigen Material hergestellt ist, derart, dass eine Betätigung des Signalschalters (29) durch das Aktivierungsteil (22) den Signalschalter (29) schließt oder öffnet.

3. Ausrichtungsindikator für eine Arzneimittelabgabevorrichtung (10) nach Anspruch 2, wobei das Aktivierungsteil (22) zwischen der ersten Position und der zweiten Position schiebend bewegbar ist.

4. Ausrichtungsindikator für eine Arzneimittelabgabevorrichtung (10) nach Anspruch 2, wobei das Aktivierungsteil (22) zwischen der ersten Position und der zweiten Position rollend bewegbar ist.

5. Ausrichtungsindikator für eine Arzneimittelabgabevorrichtung (10) nach Anspruch 2, wobei das Aktivierungsteil (22) aus einem flüssigen Leiter hergestellt ist, und wobei der flüssige Leiter zwischen der ersten Position und der zweiten Position fließen kann.

6. Ausrichtungsindikator für eine Arzneimittelabgabevorrichtung (10) nach Anspruch 5, wobei der flüssige Leiter Quecksilber ist, und wobei der flüssige Leiter und der Signalschalter (29) in einem Quecksilberschalter enthalten sind.

7. Ausrichtungsindikator für eine Arzneimittelabgabevorrichtung (10) nach einem der Ansprüche 1 bis 6, wobei das Aktivierungsteil (22) in der ersten Position durch ein manuell betriebsfähiges Arretierungsteil (30) fixiert gehalten wird, bis es freigegeben wird.

8. Ausrichtungsindikator für eine Arzneimittelabgabevorrichtung (10) nach einem der Ansprüche 1 bis 6, wobei die elektrische Schaltung (28) ferner einen manuell betriebsfähigen zweiten Schalter (60) umfasst, und wobei das Signal durch das Signalisierungselement (26) erzeugt wird, wenn der Signalschalter (29) und der zweite Schalter (60) betätigt werden.

9. Ausrichtungsindikator für eine Arzneimittelabgabevorrichtung (10) nach Anspruch 1, wobei das Signal, das durch das Signalisierungselement (26) erzeugt wird, ein akustisches Signal und/oder ein taktiles Signal und/oder ein visuelles Signal ist.

10. Ausrichtungsindikator für eine Arzneimittelabgabevorrichtung (10) nach Anspruch 1, wobei das Stützelement (20) an der Arzneimittelabgabevorrichtung (10) anbringbar ist.

11. Ausrichtungsindikator für eine Arzneimittelabgabevorrichtung (10) nach Anspruch 1, wobei das Stützelement (20) in die Arzneimittelabgabevorrichtung (10) integriert ist.

12. Ausrichtungsindikator für eine Arzneimittelabgabevorrichtung (10) nach einem der Ansprüche 1 bis 6, wobei die elektrische Schaltung (28) ebenso eine Steuereinheit und eine Kommunikationseinheit, die in der Lage ist, Ausrichtungsdaten an eine externe Vorrichtung drahtlos zu kommunizieren, umfasst, wobei die Ausrichtungsdaten von der Steuereinheit abgerufen werden.

13. Ausrichtungsindikator für eine Arzneimittelabgabevorrichtung (10) nach einem der Ansprüche 1 bis 12, wobei der Ausrichtungsindikator als ein verpacktes Modul, das an einen äußeren Teil einer Arzneimittelabgabevorrichtung (10) angebracht werden soll, aufgebaut ist.

14. Ausrichtungsindikator für eine Arzneimittelabgabevorrichtung (10) nach Anspruch 9, wobei das Signalisierungselement (26) ein positives Signal, das eine korrekte Ausrichtung anzeigt, oder ein negatives Signal, das eine falsche Ausrichtung anzeigt, erzeugen kann.

15. Arzneimittelabgabevorrichtung (10), umfassend den Ausrichtungsindikator nach einem der Ansprüche 1 bis 14.

## Revendications

1. Indicateur d'orientation destiné à un dispositif d'administration de médicament (10) ayant un axe longitudinal (A) et un organe d'administration (50) ; le dispositif d'administration de médicament (10) étant conçu pour être orienté avec son organe d'administration (50) pointant vers le haut pendant un mélange ou un amorçage, dans lequel l'indicateur d'orientation comprend :
- un élément de support (20) ayant une extrémité proximale et une extrémité distale et étant généralement allongé le long de l'axe longitudinal (A) du dispositif d'administration de médicament (10),
- un organe d'activation (22) pouvant être déplacé par rapport à l'élément de support (20), entre une première position et une seconde position,
- un circuit électrique (28) comprenant un commutateur de signal (29),
- un élément de signalisation (26) connecté au circuit électrique (28),
dans lequel l'organe d'activation (22) peut être déplacé sous la force gravitationnelle lorsque l'élément de support (20) est incliné, pour actionner le commutateur de signal (29), de telle sorte qu'un signal est généré par l'élément de signalisation (26) informant un utilisateur qu'une orientation adéquate du dispositif (10) a été atteinte pour un mélange ou un amorçage.

2. Indicateur d'orientation destiné à un dispositif d'administration de médicament (10) selon la revendication 1, dans lequel l'organe d'activation (22) est constitué d'un matériau conducteur, de telle sorte qu'un actionnement du commutateur de signal (29), par l'organe d'activation (22), ferme ou ouvre le commutateur de signal (29).

3. Indicateur d'orientation destiné à un dispositif d'administration de médicament (10) selon la revendication 2, dans lequel l'organe d'activation (22) peut être déplacé par coulissement entre la première position et la seconde position.

4. Indicateur d'orientation destiné à un dispositif d'administration de médicament (10) selon la revendication 2, dans lequel l'organe d'activation (22) peut être déplacé par roulement entre la première position et la seconde position

5. Indicateur d'orientation destiné à un dispositif d'administration de médicament (10) selon la revendication 2, dans lequel l'organe d'activation (22) est constitué d'un conducteur liquide et dans lequel le conducteur liquide peut s'écouler entre la première position et la seconde position.

6. Indicateur d'orientation destiné à un dispositif d'administration de médicament (10) selon la revendication 5, dans lequel le conducteur liquide est du mercure et dans lequel le conducteur liquide et le commutateur de signal (29) sont compris dans un commutateur à mercure.

7. Indicateur d'orientation destiné à un dispositif d'administration de médicament (10) selon l'une quelconque des revendications 1 à 6, dans lequel l'organe d'activation (22) est maintenu fixe dans la première position jusqu'à ce qu'il soit libéré par un organe de verrouillage (30) actionnable manuellement.

8. Indicateur d'orientation destiné à un dispositif d'administration de médicament (10) selon l'une quelconque des revendications 1 à 6, dans lequel le circuit électrique (28) comprend en outre un second commutateur (60) actionnable manuellement, et dans lequel le signal est généré par l'élément de signalisation (26) lorsque le commutateur de signal (29) et le second commutateur (60) sont actionnés.

9. Indicateur d'orientation destiné à un dispositif d'administration de médicament (10) selon la revendication 1, dans lequel le signal généré par l'élément de signalisation (26) est un signal audible et/ou un signal tactile et/ou un signal visuel.

10. Indicateur d'orientation destiné à un dispositif d'administration de médicament (10) selon la revendication 1, dans lequel l'élément de support (20) peut être attaché au dispositif d'administration de médicament (10).

11. Indicateur d'orientation destiné à un dispositif d'administration de médicament (10) selon la revendication 1, dans lequel l'élément de support (20) est intégré dans le dispositif d'administration de médicament (10).

12. Indicateur d'orientation destiné à un dispositif d'administration de médicament (10) selon l'une quelconque des revendications 1 à 6, dans lequel le circuit électrique (28) comprend également une unité de commande et une unité de communication capable de communiquer sans fil des données d'orientation avec un dispositif externe, ces données d'orientation étant récupérées auprès de l'unité de commande.

13. Indicateur d'orientation destiné à un dispositif d'administration de médicament (10) selon l'une quelconque des revendications 1 à 12, dans lequel l'indicateur d'orientation se présente sous la forme d'un module emballé à attacher à une partie externe d'un dispositif d'administration de médicament (10).

14. Indicateur d'orientation destiné à un dispositif d'administration de médicament (10) selon la revendication 9, dans lequel l'élément de signalisation (26) est apte à générer un signal positif, indiquant une orientation correcte ou un signal négatif, indiquant une orientation incorrecte.

15. Dispositif d'administration de médicament (10) comprenant l'indicateur d'orientation selon l'une quelconque des revendications 1 à 14.
